# EUROPEAN PATENT SPECIFICATION

(11) **EP 2 603 139 B1**
(45) Date of publication and mention of the grant of the patent: **16.08.2017**
(21) Application number: 11719835.8
(22) Date of filing: 17.05.2011
(51) Int. Cl.: A61B 5/00

(54) **CAMERA DEVICE FOR EVALUATING CONDITION OF SKIN OR HAIR**
KAMERAVORRICHTUNG ZUR BEURTEILUNG DES HAUT- ODER HAARZUSTANDS
DISPOSITIF DE TYPE APPAREIL DE PRISE DE VUES UTILISABLE EN VUE DE L'ÉVALUATION DE L'ÉTAT DE LA PEAU OU DES CHEVEUX

(30) Priority: 13.08.2010 US 855730
(43) Date of publication of application: 19.06.2013
(73) Proprietor: Unilever PLC, London, Greater London EC4P 0DY (GB); Unilever N.V., 3013 AL Rotterdam (NL)
(72) Inventor: KRISHNAN, Srinivasan, Trumbull, Connecticut 06611 (US); NICHOL, Jamie, Gordon, Arlington, Massachusetts 02476 (US); CICHOWLAS, Bruce, Framingham, MA 01701 (US)
(74) Representative: Corsten, Michael Allan
(86) International application number: PCT/EP2011/057938
(87) International publication number: WO 2012/019795

(56) References cited:
- WO-A2-2010/049907
- JP-A- 2003 210 416
- US-A1- 2007 060 819
- US-A1- 2008 221 411

## Description

### BACKGROUND OF THE INVENTION

### Field of the Invention

The invention concerns a hand holdable device for simultaneously measuring multiple parameters defining a person's skin or hair condition.

### The Related Art

Most people do not have perfect skin or hair. Imperfections can cosmetically be treated with an appropriate product. Selection of the appropriate product is often not scientific. Some will provide excellent results, others modest improvement, and still others will simply not be effective. A correct match needs to start with an evaluation of a person's skin or hair condition.

Evaluation systems have been reported in the literature. For instance, US 5 622 692 (Rigg et al.) discloses a hand-held device for measuring skin color at a point of retail sale and recommending a suitable facial foundation. US 5 945 112 (Flynn et al.) discloses a method for providing a customized skin foundation product to cover human skin imperfections. The steps include spectrophotometrically measuring a customer's normal skin to obtain normal skin coloration values of lightness, redness and yellowness. These coloration values are then converted through calculation to a modified value determined by a set program. The skin analyzing module is a hand-held spectrophotometer/colorimeter operating with at least one visible light source such as an LED operating in the 400-900 nm wavelength range.

JP 2003 210416 (Wave Cyber KK) describes a skin measuring instrument held within a transparent body. A light source shines illuminating light through the transparent body and an optical detector absorbs reflected light from the contact surface. Water content is measured by way of static capacitance at a skin contact point. Also present within the body is a camera for photographing the skin surface.

Other similar skin diagnosis devices with illuminating arrangements are WO 2010/049907 A2 or US 2007/0060819 A1. Devices to measure moisture and skin blemishes/topography are commercially available. Yet rarely is all the information collected with a single instrument. When this occurs such as in the aforementioned Japanese disclosure, coordination of camera, hydrometer and other analytics to focus on the identical small area of skin is problematic. Better devices are required which simultaneously measure/deliver information on moisture content, blemishes and topography.

### SUMMARY OF THE INVENTION

A device for evaluating skin or hair condition is provided which includes:
(i) a housing;
(ii) a hydration meter for measuring moisture, the meter being supported within the housing and having an external surface contactable against skin or hair to measure moisture content thereof, the meter including at least two metallic wires with their respective capacitance sensitive to differences in dielectric constant;
(iii) a camera held within the housing for electronically recording images of an area of skin or hair to be evaluated, the camera being supported within the housing, the electronically recorded images being received through a light transparent window at a front end of the housing; and
wherein the window is surrounded by the external surface of the hydration meter.

### BRIEF DESCRIPTION OF THE DRAWING

Further advantages and features of the present invention will become more apparent from consideration of the drawing wherein:
Fig. 1 is a perspective view of an embodiment;
Fig. 2 is an end plan view of the embodiment shown in Fig. 1;
Fig. 3 is a half-shell view of Fig. 1;
Fig. 4 is an enlarged partial view of Fig. 2 highlighting the camera and diffuser area; and
Fig. 5 a-e are a series of embodiments of alternate diffusers.

### DETAILED DESCRIPTION OF THE INVENTION

Evaluation of skin condition needs a combination of a moisture measurement and a visual analysis of the surface. These two different types of measurements must be focused on the same small area. Now we have found that a device that combines both a hydration meter and a camera can best operate in tandem via a common window. The external contact surfaces for the hydration meter circumscribe the border of the window. A camera is focused through this window thereby insuring cooperation on an identical surface area for analysis.

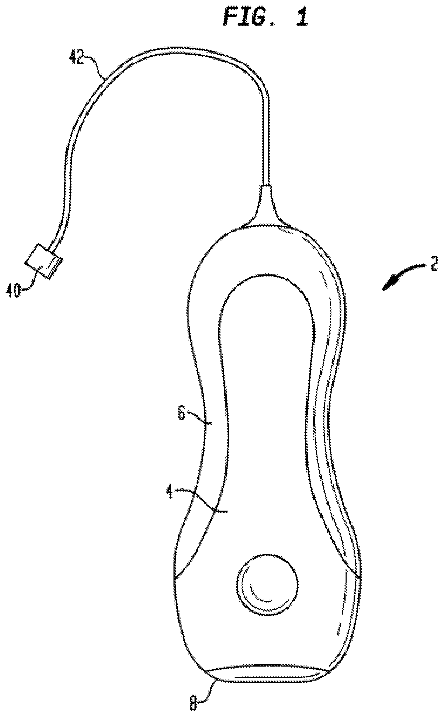

Fig. 1 illustrates one embodiment of the hand-held device. By the term "hand-holdable" or "hand-held" is meant a device measuring in length less than 35 cm, preferably between 10 and 25 cm (not including cord) and a width between 2 and 8 cm, preferably between 3 and 6 cm. The device features a housing 2 with a shell 4 and a gripping portion 6. Normally the shell is formed of a relatively hard plastic such as ABS (polyacrylonitrile- butadiene-styrene) which is a high impact resistant plastic. Advantageously the gripping area will be formed of a less rigid material such as a rubber.

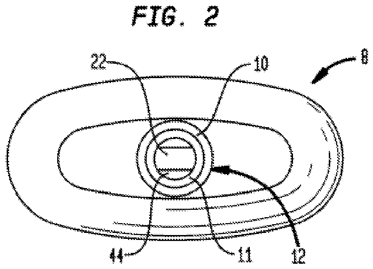

Measurements are taken from an evaluation area 8 of the device. Fig. 2 best illustrates the evaluation area. Normally area 8 is on an end of the device distant from any electrical or fiber optic cords exiting the housing. Area 8 has at least a central section formed of a transparent panel. Clear plastic is useful for this purpose. Light transmission is a necessary property for this panel.

When the evaluation area is contacted against skin, the first contact surface is a moisture sensing cell 10 of a hydration meter 12. The sensing cell 10 picks up electrical signals from the stratum corneum of skin. Metallic electrical conducting wires, preferably of copper on a circuit board, are embedded within a hardened resin of the sensing cell. These wires are sensitive to differences in dielectric constant of their aqueous environment. Differences in relative electrical capacitance resulting from differences in the dielectric constant reveal the measure of moisture at the skin or hair surface. Hydration meters are commercially available from Courage-Khazaka Electronics, Koln, Germany. The sensing cell 10 is circular (donut shaped) having a central transparent window 11 through which light can be transmitted.

Although in the preferred embodiment, the sensing cell 10 is circular, alternate configurations are possible. These configurations may be oval or polygonal. Illustrative but not limiting are polygonal structures such as square, rectangle, triangle, pentagonal, hexagonal and octagonal. Further, the circumscribing sensing cell may also have an irregular structure wherein the polygonal sides are not equivalent in length one to another (e.g. trapezoidal).

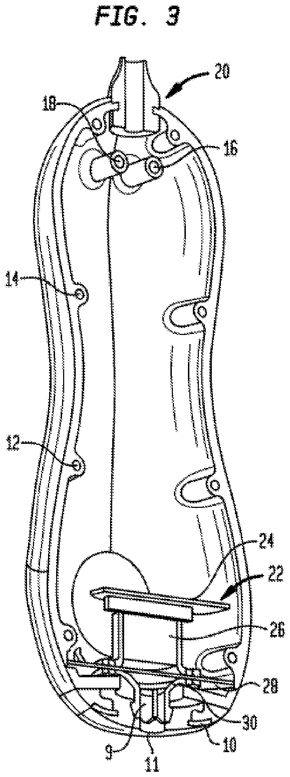

Fig. 3 is a view with half of the housing removed. For this embodiment the housing is a pair of separately molded plastic shells. These shells are fastened together by alternating male and female peg/socket clasps 12 and 14, respectively, arranged along a periphery of the shells. Also present are a male and female pair of wall joints 16 and 18, respectively, near a rear end of the housing. These wall joints project inward to engage their respective joints on each of the half shells.

A camera 22 is mounted within the housing. Components of the camera include a camera microchip 24, a lens system 26, an optical image receiver 28 and a diffuser 30. The camera microchip 24 has a circuit board electrical arrangement that can adjust the lens system, store image information and transmit the images to a receiver outside the housing.

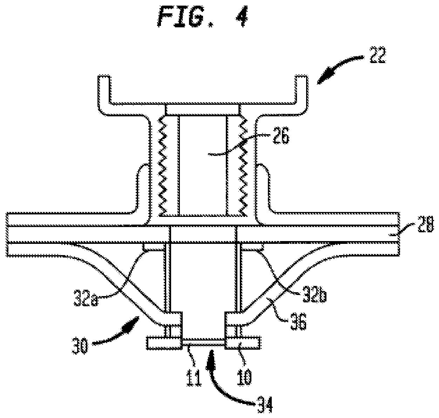

One or more light emitting diodes (LED) 32a/32b are positioned downstream from the optical image receiver 28. Fig. 4 best illustrates the arrangement. The LED may be selected from blue wavelength light emitter (to improve quality of images), from infrared wavelength light emitter and combinations thereof. In the preferred embodiment, the LEDs will be utilized to emit but not receive light.

Although the LED 32a and 32b are oriented to emit light downward to the front end 34 of the housing, their light will be intercepted by the funnel-shaped wall segment 36 of the diffuser 30. As a consequence, the LED emitted light will scatter back and forth along the diffuser and down a tunnel 9 to finally exit through the central transparent window 11 at the front end of the housing.

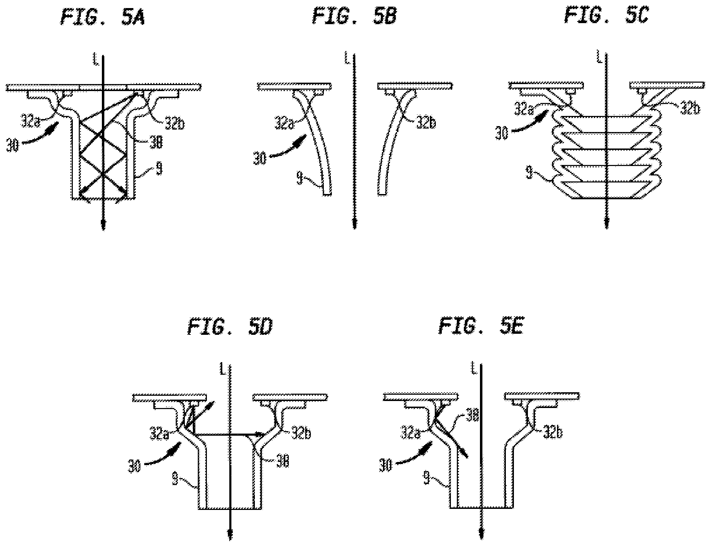

Fig. 5a provides another view of the LED, diffuser and the light path L. The emitted light 38 moves along the diffuser and tunnel in a zig- zag pattern. This pattern is achieved by the LED being spaced away from any straight bore sections of the tunnel 9 that would ordinarily allow the emitted light uninterfered transit from the LED through the central window 11. Alternate embodiments of the diffuser 30 are shown in Fig. 5b, 5c, 5d and 5e. In other embodiments of this invention, the camera may be operative without the diffuser albeit providing images of lesser definition.

Reflectance spectroscopy measurements can be confused by gloss on the skin surface. The special arrangement of the LEDs in the sensor allows measurement of surface gloss as well as the spectro signature of the skin.

Data generated from the hydration meter and the LEDs initially is in analog form. This form arises because the meter and LEDs are transducers which inherently provide an analog response to the physical event being measured. The analog data is next converted to digital values (numbers stored in a micro-controller). Thereafter, the digital values are transformed back into analog mode, this time as an audio wave signal. The audio signal is thereafter transformed back into a digital signal at a downstream USB microchip. By this manner we can double the base frequency or reduce the base frequency by half. In summary, the data is converted from analog to digital to analog to digital. This ADAD conversion is a key factor allowing use of off-the-shelf components to transfer skin data in a way the computer already recognizes.

The generated data is converted through a pre-set series of calculations to identify a Skin Index unique to the measured skin area. The Skin Index permits a user to monitor their skin over a period of time. A product recommended by the program to adjust the consumer's skin into an improved condition may be applied over the monitored period. This allows a consumer to evaluate effectiveness of the product or any other products that might be applied to improve the skin condition.

A USB port 40 is attached to an end of an electrical wire or optical fiber cord (data cord) 42 as shown in Fig. 1. the USB port can plug into a computer to access a program companion to the device. However, the system may also work by Bluetooth connectivity or serial port connection routes.

Most preferred is that power to the system be delivered externally from an electric grid. Alternatively, power can be supplied by a rechargeable battery or disposable batteries within the device. In circumstances wherein power is supplied by a rechargeable or a disposable battery, the only cord projecting from the housing is the data cord 42.

In one aspect of the invention, the device can be considered as a "personal trainer" for a user's skin.

A polarizer plate 44 may be placed in tandem with the central window. All emitted light will pass through the polarizer plate. With the polarizer, the camera better images wrinkles and spots.

The term "comprising" is meant not to be limiting to any subsequently stated elements but rather to encompass non-specified elements of major or minor functional importance. In other words the listed steps, elements or options need not be exhaustive. Whenever the words "including" or "having" are used, these terms are meant to be equivalent to "comprising" as defined above.

Except in the operating and comparative examples, or where otherwise explicitly indicated, all numbers in this description indicating amounts of material ought to be understood as modified by the word "about".

It should be noted that in specifying any range or amount, any particular upper value can be associated with any particular lower value or amount.

## Claims

1. A device for evaluating skin or hair condition comprising:
(i) a housing (2);
(ii) a hydration meter (12) for measuring moisture, the meter being supported within the housing and having an external surface contactable against skin or hair to measure moisture content thereof, the meter comprising at least two adjacent metallic wires with their capacitance sensitive to differences In dielectric constant;
(iii) a camera (22) held within the housing for electronically recording images of an area of skin or hair to be evaluated, the camera being supported within the housing, the electronically recordable images being received through a light transparent window (11) at a front end of the housing;
(iv) a diffuser (30) upstream from a tunnel (9) leading to the light transparent window, the diffuser being unitarily formed with walls of the tunnel;
(v) at least one light emitting diode (32a, 32b) arranged between the camera and the diffuser;
wherein the window is surrounded by the external surface of the hydration meter;
wherein the hydration meter and camera generate data in analog form,
wherein the data in analog form is converted to digital values and then retransformed into an analog form as an audio signal for transmission; and
wherein the at least one light emitting diode is spaced away from any straight bore sections of the tunnel that would ordinarily allow the emitted light uninterferred transit from the at least one light emitting diode through the central window thereby emitted light moves along the diffuser and tunnel in a zig-zag pattern.

2. A device according to claim 1 wherein walls of the diffuser are funnel shaped constricting inward to where the walls of the diffuser connect to the tunnel.

3. A device according to claim 1 wherein light arising from the light emitting diode travels downwardly toward the light transparent window but first is intercepted and reflected by walls of the diffuser.

4. A device according to claim 1 or claim 3 wherein the light emitting diode emits infrared wavelength light.

5. A device according to claim 1 or claim 3 wherein the light emitting diode emits blue wavelength light.

6. A device according to any one of the preceding claims further comprising a polarizing plate (44) arranged downstream from the camera.

7. A device according to any one of the preceding claims wherein the external surface of the hydration meter is circular or oval and thereby forms a respective circular or oval window.

## Patentansprüche

1. Vorrichtung zum Bewerten eines Haut- oder Haarzustands, wobei die Vorrichtung Folgendes umfasst:
(i) ein Gehäuse (2);
(ii) ein Hydratationsmessgerät (12) zum Messen von Feuchtigkeit, wobei das Messgerät in dem Gehäuse gehalten wird und eine äußere Oberfläche aufweist, die mit Haut oder Haar in Kontakt gebracht werden kann, um deren Feuchtigkeitsgehalt zu messen, wobei das Messgerät wenigstens zwei aneinander grenzende Metalldrähte umfasst, deren Kapazität auf Unterschiede in der Dielektrizitätskonstante reagiert;
(iii) eine Kamera (22), die in dem Gehäuse gehalten wird, um Bilder eines Bereichs von Haut oder Haar, der bewertet werden soll, elektronisch aufzuzeichnen, wobei die Kamera in dem Gehäuse gehalten wird, wobei die elektronisch aufzuzeichnenden Bilder durch ein lichtdurchlässiges Fenster (11) an einem vorderen Ende des Gehäuses aufgenommen werden;
(iv) einen Diffusor (30), der einer Röhre (9) vorgeschaltet ist, die zu dem lichtdurchlässigen Fenster führt, wobei der Diffusor mit Wänden der Röhre einteilig ausgebildet ist;
(v) wenigstens eine Leuchtdiode (32a, 32b), die zwischen der Kamera und dem Diffusor angeordnet ist;
wobei das Fenster von der äußeren Oberfläche des Hydratationsmessgeräts umgeben ist;
wobei das Hydratationsmessgerät und die Kamera Daten in analoger Form erzeugen,
wobei die Daten in analoger Form in digitale Werte umgesetzt werden und dann erneut in eine analoge Form als ein Audiosignal zur Übertragung zurückverwandelt werden; und
wobei die wenigstens eine Leuchtdiode von irgendwelchen geraden Bohrabschnitten der Röhre beabstandet ist, die gewöhnlich zulassen würden, dass das emittierte Licht von der wenigstens einen Leuchtdiode ungestört durch das zentrale Fenster treten würde, wobei sich das emittierte Licht entlang des Diffusors und der Röhre in einem Zickzackmuster bewegt.

2. Vorrichtung nach Anspruch 1, wobei die Wände des Diffusors trichterförmig sind, wobei sie dort nach innen enger werden, wo die Wände des Diffusors mit der Röhre verbunden sind.

3. Vorrichtung nach Anspruch 1, wobei sich Licht, das von der Leuchtdiode ausgeht, abwärts in Richtung des lichtdurchlässigen Fensters bewegt, jedoch zuerst von Wänden des Diffusors aufgenommen und reflektiert wird.

4. Vorrichtung nach Anspruch 1 oder Anspruch 3, wobei die Leuchtdiode Licht im Infrarot-Wellenlängenbereich emittiert.

5. Vorrichtung nach Anspruch 1 oder Anspruch 3, wobei die Leuchtdiode Licht im blauen Wellenlängenbereich emittiert.

6. Vorrichtung nach einem der vorhergehenden Ansprüche, die ferner eine Polarisationsplatte (44) umfasst, die der Kamera nachgeschaltet angeordnet ist.

7. Vorrichtung nach einem der vorhergehenden Ansprüche, wobei die äußere Oberfläche des Hydratationsmessgeräts kreisförmig oder oval ist und dadurch ein entsprechendes kreisförmiges oder ovales Fenster bildet.

## Revendications

1. Dispositif pour évaluer l'état de la peau ou des cheveux comprenant :
(i) un logement (2) ;
(ii) un dispositif de mesure d'hydratation (12) pour mesurer l'humidité, le dispositif de mesure étant supporté dans le logement et comportant une surface externe qui peut être mise en contact avec la peau ou les cheveux pour mesurer la teneur en humidité de ceux-ci, le dispositif de mesure comprenant au moins deux fils métalliques adjacents dont la capacitance est sensible aux différences de constante diélectrique ;
(iii) une caméra (22) contenue dans le logement pour enregistrer électroniquement des images d'une zone de peau ou de cheveux à évaluer, la caméra étant supportée dans le logement, les images enregistrables électroniquement étant reçues à travers une fenêtre transparente à la lumière (11) à une extrémité avant du logement ;
(iv) un diffuseur (30) en amont d'un tunnel (9) menant à la fenêtre transparente à la lumière, le diffuseur étant formé de manière unitaire avec les parois du tunnel ;
(v) au moins une diode électroluminescente (32a, 32b) agencée entre la caméra et le diffuseur ;
dans lequel la fenêtre est entourée par la surface externe du dispositif de mesure d'hydratation ;
dans lequel le dispositif de mesure d'hydratation et la caméra génèrent des données sous forme analogique,
dans lequel les données sous forme analogique sont converties en des valeurs numériques et retransformées ensuite en une forme analogique en tant que signal audio pour transmission ; et
dans lequel ladite au moins une diode électroluminescente est espacée des sections d'alésage droites du tunnel qui permettraient normalement le transit sans interférence de la lumière émise de ladite au moins une diode électroluminescente à travers la fenêtre centrale, la lumière émise se propageant de ce fait le long du diffuseur et du tunnel en un motif en zigzag.

2. Dispositif selon la revendication 1, dans lequel les parois du diffuseur sont en forme d'entonnoir qui se resserre vers l'intérieur jusqu'à l'endroit où les parois du diffuseur rejoignent le tunnel.

3. Dispositif selon la revendication 1, dans lequel la lumière provenant de la diode électroluminescente se propage vers le bas vers la fenêtre transparente à la lumière, mais est d'abord interceptée et réfléchie par les parois du diffuseur.

4. Dispositif selon la revendication 1 ou la revendication 3, dans lequel la diode électroluminescente émet une lumière dans les infrarouges.

5. Dispositif selon la revendication 1 ou la revendication 3, dans lequel la diode électroluminescente émet une lumière dans les bleus.

6. Dispositif selon l'une quelconque des revendications précédentes comprenant en outre une plaque de polarisation (44) agencée en aval de la caméra.

7. Dispositif selon l'une quelconque des revendications précédentes, dans lequel la surface externe du dispositif de mesure d'hydratation est circulaire ou ovale et forme, de ce fait, une fenêtre circulaire ou ovale respective.
